(19) Europäisches Patentamt
European Patent Office
Office européen des brevets

(11) **EP 2 365 322 A1**

(12) **EUROPEAN PATENT APPLICATION**

(43) Date of publication:
**14.09.2011 Bulletin 2011/37**

(51) Int Cl.:
**G01N 27/02** *(2006.01)*    **G01N 27/416** *(2006.01)*

(21) Application number: **11250176.2**

(22) Date of filing: **15.02.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **16.02.2010  US 706097**

(71) Applicant: **Hamilton Sundstrand Corporation
Windsor Locks, CT 06096-1010 (US)**

(72) Inventor: **Lou, Yiming
Diamond Bar, California 91765 (US)**

(74) Representative: **Tomlinson, Kerry John
Dehns
St Bride's House
10 Salisbury Square
London
EC4Y 8JD (GB)**

(54)    **Gas sensor age compensation and failure detection**

(57)    A method, system (100), and computer program product for age compensation for a gas sensor (101) are provided. The method includes determining an impedance spectrum for the gas sensor (101) by an impedance analyzer (103); determining a set of principal parameters based on the impedance spectrum by a principal parameter identifier (104); constructing an age compensation model for the gas sensor (101) using the set of principal parameters by an age compensation modeler (105); and applying the age compensation model to an output of the gas sensor (10 1). Gas sensor (101) failure detection is also provided.

FIG. 1

EP 2 365 322 A1

**Description**

FIELD OF INVENTION

The subject matter disclosed herein relates generally to the field of gas sensor monitoring.

DESCRIPTION OF RELATED ART

An electrochemical gas sensor may comprise an amperometric sensor, comprising a working (or sensing) electrode, a counter electrode and an electrolyte. The sensor may also include a reference electrode. The electrochemical gas sensor comprises an electrochemical cell, which includes the electrodes and an electrolyte. In operation, the target gas (i.e., the gas the electrochemical gas sensor is designed to detect) reacts with the surface of the sensing electrode, and a current proportional to the gas concentration is generated by the surface reaction. The current may be measured to determine the concentration of the target gas.

A major challenge of using electrochemical sensors for gas detection is senor aging, which, if not appropriately compensated, may result in significant errors in sensor readings. Scheduled routine sensor calibration using standard calibration gases may be used to maintain sensor accuracy. While this approach is standard in many industrial applications where scheduled routine sensor calibration may be performed by maintenance personnel, it is not feasible in situations such as space flight where there is no room for the storage of the standard gases required to calibrate the sensor. Further, the sensor may eventually fail, leading to undetected unacceptable target gas concentrations; this is especially problematic if the target gas is toxic.

BRIEF SUMMARY

According to one aspect of the invention, a method for age compensation for a gas sensor includes determining an impedance spectrum for the gas sensor by an impedance analyzer; determining a set of principal parameters based on the impedance spectrum by a principal parameter identifier; constructing an age compensation model for the gas sensor using the set of principal parameters by an age compensation modeler; and applying the age compensation model to an output of the gas sensor.

According to another aspect of the invention, a system for age compensation for a gas sensor includes an impedance analyzer configured to determine an impedance spectrum for the gas sensor; a principal parameter identifier configured to determine a set of principal parameters based on the impedance spectrum; and an age compensation modeler configured to construct an age compensation model for the gas sensor using the set of principal parameters, and to apply the age compensation model to an output of the gas sensor.

According to another aspect of the invention, a computer program product comprises a computer readable storage medium containing computer code that, when executed by a computer, implements a method for age compensation and failure detection for a gas sensor, wherein the method includes determining an impedance spectrum for the gas sensor; determining a set of principal parameters based on the impedance spectrum; constructing an age compensation model for the gas sensor using the set of principal parameters; and applying the age compensation model to an output of the gas sensor.

Systems, methods, and a computer program product for gas sensor failure detection are also provided.

Other aspects, features, and techniques of the invention will become more apparent from the following description taken in conjunction with the drawings.

BRIEF DESCRIPTION OF THE SEVERAL VIEWS OF THE DRAWINGS

Referring now to the drawings wherein like elements are numbered alike in the several FIGURES:

FIG. 1 illustrates an embodiment of a system for gas sensor age compensation and failure detection.

FIG. 2 illustrates an embodiment of a method for gas sensor age compensation.

FIG. 3 illustrates an embodiment of a Randles equivalent circuit.

FIG. 4 illustrates an embodiment of a method for gas sensor failure detection.

FIG. 5 illustrates a computer that may be used in conjunction with embodiments of systems and methods for gas sensor age compensation and failure detection.

DETAILED DESCRIPTION

Embodiments of systems and methods for gas sensor age compensation and failure detection are provided, with exemplary embodiments being discussed below in detail. An age compensation model for an electrochemical gas sensor may be constructed using sensor impedance spectrum data, and a compensation signal may be generated from the age compensation model to automatically compensate for sensor drift due to aging. Imminent failure of the electrochemical gas sensor may also be automatically detected, so that a warning message may be issued before the actual occurrence of sensor failure.

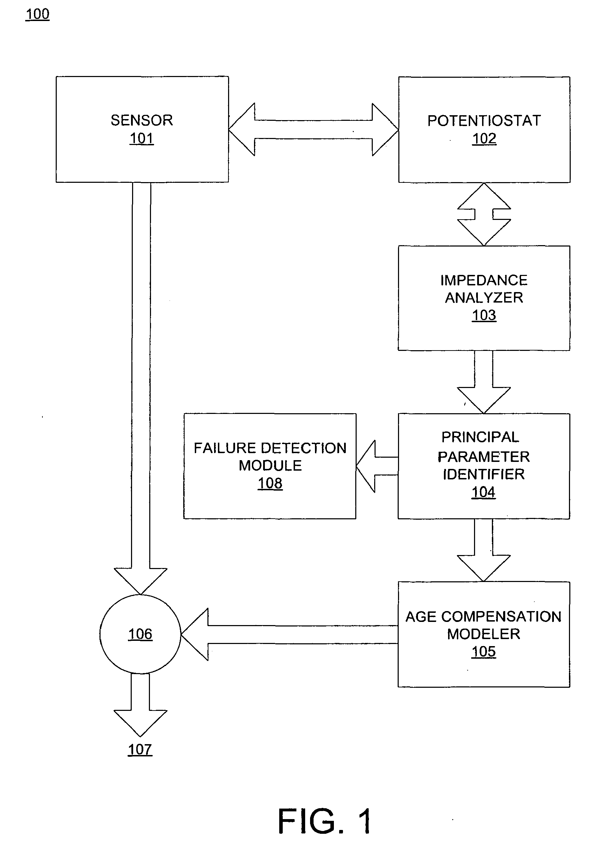

FIG. 1 illustrates an embodiment of a system 100 for gas sensor age compensation and failure detection. Sensor 101 may comprise any appropriate gas sensor, including but not limited to a carbon monoxide (CO) or a hydrogen cyanide (HCN) sensor. Sensor 101 is in communication with potentiostat 102, which gathers impedance data from sensor 101. Potentiostat 102 sends the gathered impedance data to impedance analyzer 103. Failure detection module 108, principal parameter identifier 104, and age compensation modeler 105 receive data from impedance analyzer 103. A signal is generated based on the age compensation model determined by age compensation modeler 105, and the signal is added to the output from sensor 101 at adder 106 to give age compensated sensor output 107.

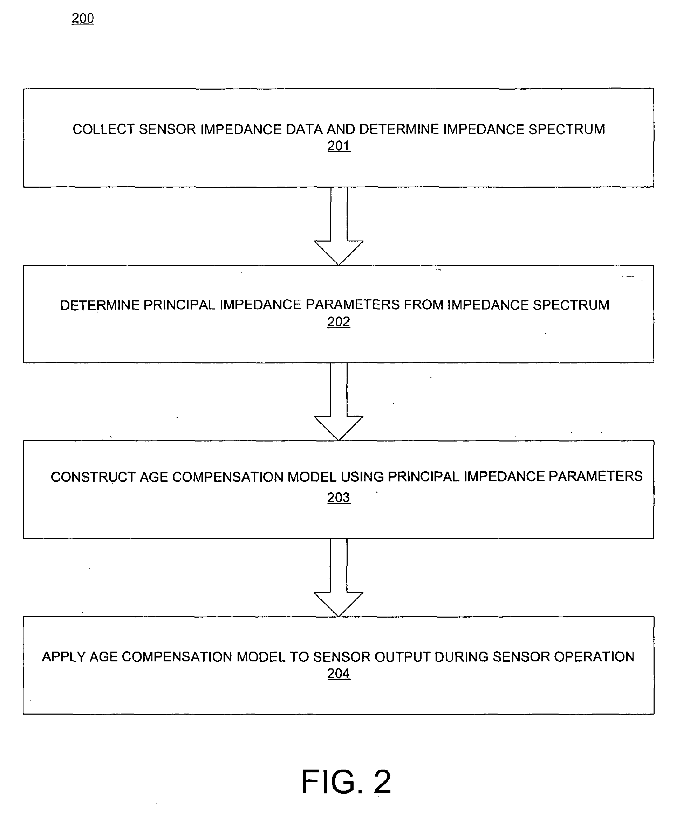

FIG. 2 illustrates an embodiment of a method 200 for sensor age compensation. FIG. 2 is discussed with reference to FIG. 1. In block 201, sensor impedance data is collected and the impedance spectrum is determined. In block 202, principal impedance parameters are determined for the sensor using the impedance data. In block 203, an age compensation model is constructed. In block 204, a compensation signal for the sensor is generated from the age compensation model and applied to the sensor output. The above steps are discussed below in further detail.

In block 201, the impedance spectrum of a sensor 101 is measured by potentiostat 102. Impedance is a measurement of the ability of a circuit to resist the flow of an alternating current (AC). The electrochemical impedance of the sensor 101 may be obtained by applying an AC potential to sensor 101 and measuring the current through the sensor 101. When a very small sinusoidal potential excitation signal with frequency $f$ and amplitude $E_0$ is applied to the sensor 101, the response may be pseudo-linear. The current response is shifted in phase by $\varphi$ and has different amplitude, $I_0$. An impedance value is a complex number expressing the magnitude ($Z_0 = E_0/I_0$) and the phase shift ($\varphi$) as shown in Eq. 1:

$$E(t) = E_0 \exp(j2\pi ft)$$
$$I(t) = I_0 \exp\left[j\left(2\pi ft - \varphi\right)\right] \tag{1}$$
$$Z = \frac{E}{I} = \frac{E_0}{I_0} \exp(j\varphi) = Z_0\left(\cos\varphi + j\sin\varphi\right)$$

The impedance spectrum for the sensor 101 is obtained by measuring the impedance of the sensor 101 at a range of frequencies. This may be performed by impedance analyzer 103 using potentiostat 102. Potentiostat 102 may comprise any commercially available potentiostat in some embodiments. Sensor 101 comprises an electrochemical cell, which is a complex system involving a variety of transport-reaction processes during its operation. Important elements that may contribute to the sensor performance include electrode double-layer capacitance, electrode kinetics, diffusion layer, and electrolyte solution resistance. The interactions among the underlying physico-chemical processes also affect the characteristics of the impedance spectrum of the cell.

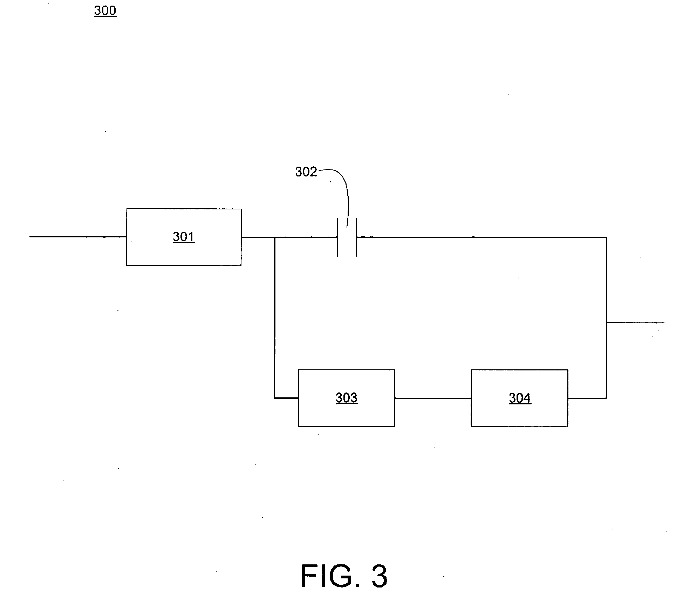

Once the impedance spectrum for sensor 101 is determined by impedance analyzer 103 in block 201, in block 202, the principal parameter identifier 104 may derive four major parameters from the impedance spectrum to characterize the sensor 101: electrolyte solution resistance ($R_s$), double-layer capacitance ($C_{dl}$), charge transfer resistance ($R_{ct}$) and Warburg impedance ($W$). A Randles equivalent circuit, an example of which is shown in FIG. 3, may be used by the principal parameter identifier 104 to determine these four parameters. Randles equivalent circuit 300 comprises solution resistance 301, double-layer capacitance 302, charge transfer resistance 303, and Warburg impedance 304. However, Randles equivalent circuit 300 is shown for illustrative purposes only; any appropriate circuit configuration may be used to determine the principal parameters. Nonlinear least squares fitting may be used to determine the values of the parameters by fitting the impedance data to the Randles equivalent circuit model. A set of optimum parameters may be obtained by minimizing the deviation between the impedance spectrum of the Randles circuit and the test data.

While there are four cell parameters that may be determined by principal parameter identifier 104 using the Randles equivalent circuit model, only a subset of the four model parameters may have a strong correlation to the sensitivity of sensor 101. Therefore, principal parameter identifier 104 may determine the principal parameters that have strong correlation to the sensor sensitivity. A principal parameter is one that accounts for a significant portion of the variability of the output signal of sensor 101.

First, the sensor sensitivity, S, may be written as a general function of the four cell parameters, as shown in Eq. 2:

$$S = f(R_s, R_{ct}, C_{dl}, W) \tag{2}$$

Whether the dependence of S on any one of the four parameters is strong or weak can be measured by comparing the partial derivatives, $\partial f/\partial R_s$, $\partial f/\partial R_{ct}$, $\partial f/\partial C_{dl}$, and $\partial f/\partial W$. If there are $N$ measurements available for $S$, $R_s$, $R_{ct}$, $C_{dl}$ and W, Eq. 2 may be written in the following form for each measurement, as shown in Eq. 3:

$$S_i = f(R_{s,i}, R_{ct,i}, C_{dl,i}, W_i) \quad i = 1, 2, \cdots N \tag{3}$$

where the subscript, $i$, indicates the $i$th measurement of the corresponding parameter. Then, the difference equations shown in Eq. 4 may be derived from Eq. 3:

$$\begin{bmatrix} \Delta S_1 \\ \Delta S_2 \\ \vdots \\ \Delta S_{N-1} \end{bmatrix} = \cdot \begin{bmatrix} \Delta R_{s,1} & \Delta R_{ct,1} & \Delta C_{dl,1} & \Delta W_1 \\ \Delta R_{s,2} & \Delta R_{ct,2} & \Delta C_{dl,2} & \Delta W_2 \\ \vdots & \vdots & \cdots & \vdots \\ \Delta R_{s,N-1} & \Delta R_{ct,N-1} & \Delta C_{dl,N-1} & \Delta W_{N-1} \end{bmatrix} \cdot \begin{bmatrix} \dfrac{\partial f}{\partial R_s} \\ \dfrac{\partial f}{\partial R_{ct}} \\ \dfrac{\partial f}{\partial C_{dl}} \\ \dfrac{\partial f}{\partial W} \end{bmatrix} \tag{4}$$

where

$$\begin{aligned} \Delta S_i &= S_{i+1} - S_i \\ \Delta R_{s,i} &= R_{s,i+1} - R_{s,i} \\ \Delta R_{ct,i} &= R_{ct,i+1} - R_{ct,i} \ ; \quad i = 1, 2, \cdots N-1 \\ \Delta C_{dl,i} &= C_{dl,i+1} - C_{dl,i} \\ \Delta W_i &= W_{i+1} - W_i \end{aligned} \tag{5}$$

Finally, the vector, $[\partial f/\partial R_s \ \partial f/\partial R_{ct} \ \partial f/\partial C_{dl} \ \partial f/\partial W]^T$ in Eq. 4 can be estimated using linear regression as shown in Eq. 6:

$$\begin{bmatrix} \dfrac{\partial f}{\partial R_s} & \dfrac{\partial f}{\partial R_{ct}} & \dfrac{\partial f}{\partial C_{dl}} & \dfrac{\partial f}{\partial W} \end{bmatrix}^T = \left(A^T A\right)^{-1} A^T y \tag{6}$$

where

$$A = \begin{bmatrix} \Delta R_{s,1} & \Delta R_{ct,1} & \Delta C_{dl,1} & \Delta W_1 \\ \Delta R_{s,2} & \Delta R_{ct,2} & \Delta C_{dl,2} & \Delta W_2 \\ \vdots & \vdots & \cdots & \vdots \\ \Delta R_{s,N-1} & \Delta R_{ct,N-1} & \Delta C_{dl,N-1} & \Delta W_{N-1} \end{bmatrix} \tag{7}$$

$$y = \begin{bmatrix} \Delta S_1 & \Delta S_2 & \cdots & \Delta S_{N-1} \end{bmatrix}^T$$

After the estimation of the vector, $[\partial f/\partial R_s \ \partial f/\partial R_{ct} \ \partial f/\partial C_{dl} \ \partial f/\partial W]^T$, the contribution to $\Delta S_i$ from the each of the parameters $R_s$, $R_{ct}$, $C_{dl}$ and $W$ may be subsequently computed as shown in Eq. 8:

$$\chi_{Rs} = \left| \frac{\partial f}{\partial R_s} \right| \cdot \sum_{i=1}^{N-1} \Delta R_{s,i}^2; \quad \chi_{Rct} = \left| \frac{\partial f}{\partial R_{ct}} \right| \cdot \sum_{i=1}^{N-1} \Delta R_{ct,i}^2$$

$$\chi_{Cdl} = \left| \frac{\partial f}{\partial C_{dl}} \right| \cdot \sum_{i=1}^{N-1} \Delta C_{dl,i}^2; \quad \chi_W = \left| \frac{\partial f}{\partial W} \right| \cdot \sum_{i=1}^{N-1} \Delta W_i^2 \tag{8}$$

Eq. 8 may be re-written as shown in Eq. 9 to compute the contributions from different parameters in percentage:

$$\chi_T = \chi_{Rs} + \chi_{Rct} + \chi_{Cdl} + \chi_W \tag{9}$$

$$\gamma_{Rs} = \chi_{Rs} / \chi_T \times 100\%; \quad \gamma_{Rct} = \chi_{Rct} / \chi_T \times 100\%$$

$$\gamma_{Cdl} = \chi_{Cdl} / \chi_T \times 100\%; \quad \gamma_W = \chi_W / \chi_T \times 100\%$$

Only the parameters associated with a significant contribution to the sensitivity of sensor 101 above a predetermined threshold may be used to construct the age compensation model. In some embodiments, a combinational contribution of 90% may be used as a criterion to select one or more principal parameters.

After the principal parameters are identified in block 202 of FIG. 2 by principal parameter identifier 104, an age compensation model may be constructed in block 203 by age compensation modeler 105. A model equation with unknown parameters is used for the construction of a nonlinear age compensation model. The equation may be based on available sensor data as well as the observations of the sensor response. The model equation may be represented as shown in Eq. 10:

$$S = g(x, \theta) \tag{10}$$

where $S$ is the sensor sensitivity, $x$ is the vector for the principal parameters and $\theta$ is the vector for model parameters.

If there are $n$ observations available for the sensor, e.g., $S_i$ and $x_i$ are available, the model parameter vector, $\theta$, can be determined by solving the optimization problem shown in Eq. 11:

$$\hat{\theta} = \min_{\theta} \left\| \bar{S} - G(x, \theta) \right\| \tag{11}$$

where $\bar{S} = [S_1 \ S_2 \ \cdots \ S_n]^T$ and $G(x,\theta) = [g(x_1,\theta) \ g(x_2,\theta) \ \cdots \ g(x_n,\theta)]^T$. $\|\cdot\|$ represents a form of vector norm.

If only one principal parameter was identified in block 202 of FIG. 2, a one-dimensional (1-D) age compensation model is determined by age compensation modeler 105. The 1-D model determines the sensitivity of the sensor 101 based on one principal parameter derived from the impedance spectrum, and is shown in Eq. 12:

$$S = a \cdot x + b \qquad (12)$$

where $S$ is the sensor sensitivity, $x$ is the cell parameter derived from the cell impedance ($R_s$, $R_{ct}$, $C_{dl}$ or $W$) that is identified as the principal component using the procedure in block 202. The parameters of the age compensation model, $a$ and $b$, are determined based on the test data gathered from the sensor 101. Linear regression may be used to estimate the parameters, $\theta = [a\ b]^T$ as shown in Eq. 13:

$$\theta = \left(X^T X\right)^{-1} X^T Y \qquad (13)$$

where

$$X = \begin{bmatrix} x_1 & x_2 & \cdots & x_N \\ 1 & 1 & \cdots & 1 \end{bmatrix}^T \qquad (14)$$

$$Y = \begin{bmatrix} S_1 & S_2 & \cdots & S_N \end{bmatrix}^T$$

In Eq. 14, $x_1, x_2, ..., x_N$ are the measurements of the cell parameter and $S_1$, $S_2$, ..., $S_N$ are the measurements of the sensor sensitivity.

If two principal parameters were identified in block 202 of FIG. 2, a 2-D age compensation model is determined by age compensation modeler 105. The 2-D age compensation model determines the sensitivity of the gas sensor based on two principal parameters derived from the impedance spectrum, and is shown in Eq. 15:

$$S = a \cdot x_1 + bx_2 + c \qquad (15)$$

where $S$ is the sensor sensitivity, $x_1$, and $x_2$ are the two cell parameters derived from the cell impedance ($R_s$, $R_{ct}$, $C_{dl}$ or $W$) that are identified as the principal components using the procedure described in block 202. The parameters of the 2-D age compensation model, $a$, $b$ and $c$, are determined based on the test data gathered from the sensor 101. Linear regression may be used to estimate the parameters, $\chi = [a\ b\ c]^T$ as shown in Eq. 16:

$$\chi = \left(\overline{X}^T \overline{X}\right)^{-1} \overline{X}^T Y \qquad (16)$$

where

$$\overline{X} = \begin{bmatrix} x_{1,1} & x_{1,2} & \cdots & x_{1,N} \\ x_{2,1} & x_{2,2} & \cdots & x_{2,N} \\ 1 & 1 & \cdots & 1 \end{bmatrix}^{T}$$

$$Y = \begin{bmatrix} S_1 & S_2 & \cdots & S_N \end{bmatrix}^{T} \tag{17}$$

In Eq. 17, $x_{1,1}$, $x_{1,2}$, ..., $x_{1,N}$ are the $N$ available measurements of the cell parameter $x_1$; $x_{2,1}$, $x_{2,2}$, ..., $x_{2,N}$ are the $N$ available measurements of the cell parameter $x_2$; and $S_1$, $S_2$, ..., $S_N$ are the $N$ available measurements of the sensor sensitivity.

If more than two principal parameters were identified in block 202 of FIG. 2, a higher-order age compensation model may be constructed by age compensation modeler 105 by following the methodology described above. The resulting age compensation model determines the sensitivity of the sensor 101 based on k cell parameters derived from the impedance spectrum, and is shown in Eq. 18:

$$S = a_1 \cdot x_1 + a_2 x_2 + \cdots + a_k x_k + a_{k+1} \tag{18}$$

where $S$ is the sensor sensitivity, $x_1$, $x_2$, ..., and $x_k$ are the $k$ cell parameters identified as the principal components using the procedure described in block 202. The parameters of the high-order age compensation model, $\eta = [a_1\ a_2\ ...\ a_k]^T$ are determined based on the test data gathered from the sensor 101 and using linear regression as shown in Eq. 19:

$$\eta = \left( \widetilde{X}^T \widetilde{X} \right)^{-1} \widetilde{X}^T Y \tag{19}$$

where

$$\widetilde{X} = \begin{bmatrix} x_{1,1} & x_{1,2} & \cdots & x_{1,N} \\ x_{2,1} & x_{2,2} & \cdots & x_{2,N} \\ \vdots & \vdots & \vdots & \vdots \\ x_{k,1} & x_{k,2} & \cdots & x_{k,N} \\ 1 & 1 & \cdots & 1 \end{bmatrix}^{T}$$

$$Y = \begin{bmatrix} S_1 & S_2 & \cdots & S_N \end{bmatrix}^{T} \tag{20}$$

In Eq. 20, $x_{i,1}$, $x_{i,2}$, ..., $x_{i,N}$ ($i=1, 2, ..., k$) are the $N$ available measurements of the cell parameter $x_i$; and $S_1$, $S_2$, ..., $S_N$ are the $N$ available measurements of the sensor sensitivity. Note that for k=1 or k=2, Eq. 18 is equivalent to Eq. 12 or Eq. 15, respectively.

Once an age compensation model is constructed by age compensation modeler 105 in block 203 of FIG. 2, the determined age compensation model is applied to the output of sensor 101 during sensor operation in block 204. The impedance spectrum and the values of the principal impedance parameters for sensor 101 may change as sensor 101 ages. Therefore, the age compensation model may be applied to sensor 101 by remeasuring the impedance of sensor 101 during operation by potentiostat 102 and impedance analyzer 103, recalculating the principal impedance parameter(s) by principal parameter identifier 104 based on the remeasured impedance spectrum provided by impedance analyzer 103, and plugging the recalculated principal impedance parameter(s) from principal parameter identifier 104 into the age compensation model determined by age compensation modeler in block 203 to compute the current sensor sensitivity. A signal may be generated based on the current sensor sensitivity determined from the age compensation model, and added to the sensor output by adder 106 to obtain age compensated sensor output 107.

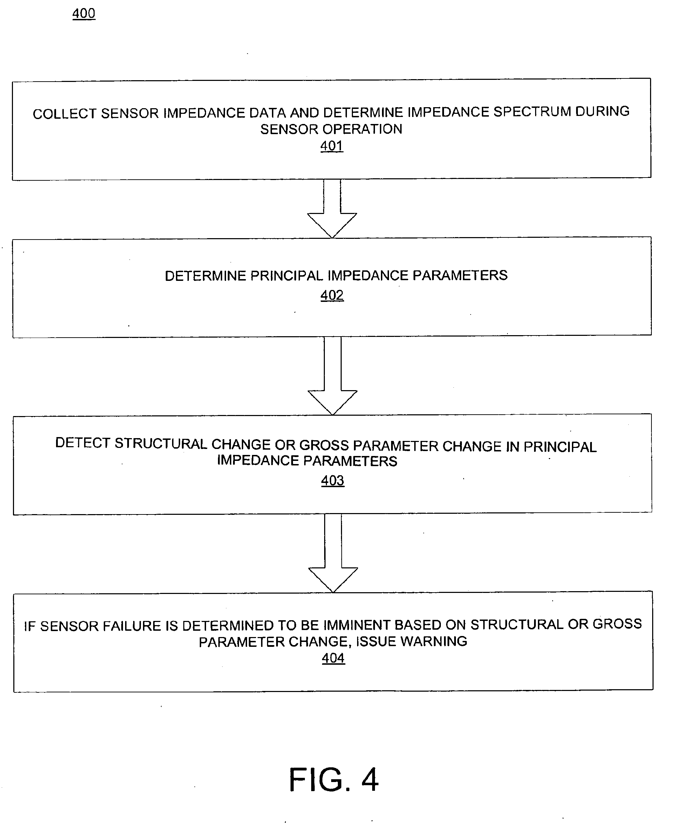

FIG. 4 illustrates an embodiment of a method 400 for sensor failure detection. FIG. 4 is discussed with reference to FIG. 1. In block 401, sensor impedance data is collected during operation of the sensor 101 by potentiostat 102 and impedance analyzer 103. In block 402, principal impedance parameters are determined by principal parameter identifier 104 using the impedance spectrum data from impedance analyzer 103. The impedance parameters are obtained in the same manner discussed above with respect to block 202 of FIG. 2.

In block 403, a structural change or gross parameter change in the sensor impedance parameters are identified by failure detection module 108. An estimation error associated with each parameter may also be obtained from the optimization-based fitting algorithm. A significant increase in the estimation error of an impedance parameter may indicate a structural change. A detected structural change may indicate an imminent sensor failure. Additionally, a significant change in the value of a parameter, i.e., a gross model parameter change, may also indicate an imminent sensor failure. A structural change in a principal parameter may be determined by comparing an estimation error associated with the parameter with a respective predetermined threshold value. A gross change in a principal parameter may be determined by comparing the parameter itself with a respective predetermined threshold value. If either the parameter or its estimation error are determined to be above their respective predetermined threshold value, in block 404, failure detection module 108 determines that a sensor failure is imminent based on the structural change or gross parameter change detected in the sensor impedance data, and issues a warning. Method 400 and block 204 of FIG. 2 may be performed contemporaneously during operation of sensor 101 by system 100.

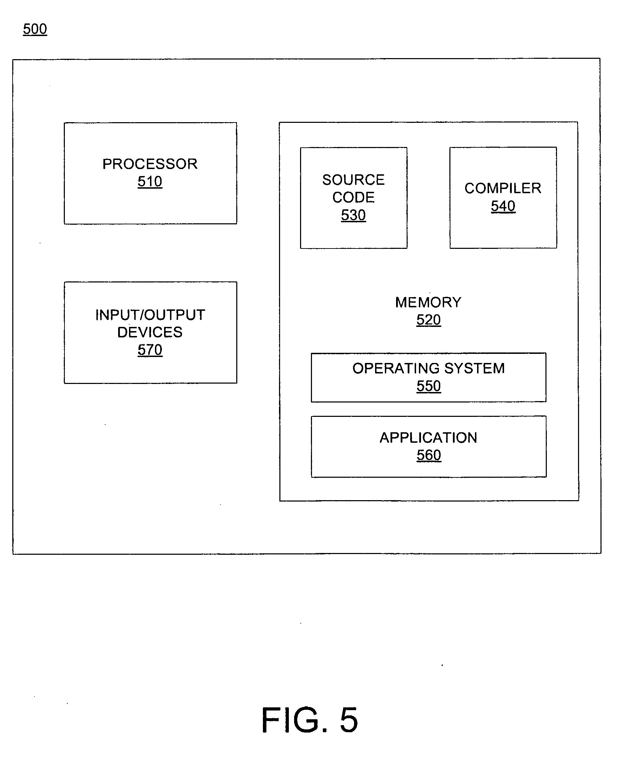

FIG. 5 illustrates an example of a computer 500 which may be utilized by exemplary embodiments of systems and methods for gas sensor age compensation and failure detection as embodied in software. Various operations discussed above may utilize the capabilities of the computer 500. One or more of the capabilities of the computer 500 may be incorporated in any element, module, application, and/or component discussed herein.

The computer 500 includes, but is not limited to, PCs, workstations, laptops, PDAs, palm devices, servers, storages, application-specific systems, embedded devices, and the like. Generally, in terms of hardware architecture, the computer 500 may include one or more processors 510, memory 520, and one or more input and/or output (I/O) devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a macroprocessor.

The memory 520 can include any one or combination of volatile memory elements (e.g., random access memory (RAM), such as dynamic random access memory (DRAM), static random access memory (SRAM), etc.) and nonvolatile memory elements (e.g., ROM, erasable programmable read only memory (EPROM), flash memory, electronically erasable programmable read only memory (EEPROM), programmable read only memory (PROM), tape, compact disc read only memory (CDROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 may include a suitable operating system (O/S) 550, compiler 540, source code 530, and one or more applications 560 in accordance with exemplary embodiments. Alternatively, the compiler 540 and source code 530 can reside on a separate development computer (not depicted). As illustrated, the application 560 comprises numerous functional components for implementing the features and operations of the exemplary embodiments. The application 560 of the computer 500 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. The application 560 for implementing exemplary embodiments may be applicable on all commercially available operating systems, as well as custom developed operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure or function-based programming language, which has routines, subroutines, and/or functions, for example but not limited to, C, C++, C#, Pascal, BASIC, API calls, HTML, XHTML, XML, ASP scripts, FORTRAN, COBOL, Perl, Java, ADA, .NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 can also include components for communicating over various networks, such as the Internet or intranet.

When the computer 500 is in operation, the processor 510 is configured to execute software stored within the memory 520, to communicate data to and from the memory 520, and to generally control operations of the computer 500 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. A "computer-readable medium" can be any means that can store, communicate, propagate, or transport the program for use by or in connection with the instruction execution system, apparatus, or device, such as memory 520 or a medium interfacing with I/O devices 570. The computer readable medium can be, for example but not limited to, an electronic, magnetic, optical, electromagnetic, infrared, or semiconductor system, apparatus, device, or propagation medium.

More specific examples (a nonexhaustive list) of the computer-readable medium may include the following: an electrical connection (electronic) having one or more wires, a portable computer diskette (magnetic or optical), a random access memory (RAM) (electronic), a read-only memory (ROM) (electronic), an erasable programmable read-only memory (EPROM, EEPROM, or Flash memory) (electronic), an optical fiber (optical), and a portable compact disc memory (CDROM, CD R/W) (optical). Note that the computer-readable medium could even be paper or another suitable medium, upon which the program is printed or punched, as the program can be electronically captured, via for instance optical scanning of the paper or other medium, then compiled, interpreted or otherwise processed in a suitable manner if necessary, and then stored in a computer memory.

In exemplary embodiments, where the application 560 is implemented in hardware, the application 560 can be implemented with any one or a combination of the following technologies, which are well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), etc.

The technical effects and benefits of exemplary embodiments include reduction of errors in the output of a electrochemical gas sensor due to aging, and early detection of possible gas sensor failure.

The terminology used herein is for the purpose of describing particular embodiments only and is not intended to be limiting of the invention. While the description of the present invention has been presented for purposes of illustration and description, it is not intended to be exhaustive or limited to the invention in the form disclosed. Many modifications, variations, alterations, substitutions, or equivalent arrangement not hereto described will be apparent to those of ordinary skill in the art without departing from the scope of the invention. Additionally, while various embodiments of the invention have been described, it is to be understood that aspects of the invention may include only some of the described embodiments. Accordingly, the invention is not to be seen as limited by the foregoing description, but is only limited by the scope of the appended claims.

## Claims

1. A method for age compensation for a gas sensor (101), the method comprising:

   determining an impedance spectrum for the gas sensor (101) by an impedance analyzer (103);
   determining a set of principal parameters based on the impedance spectrum by a principal parameter identifier (104);
   constructing an age compensation model for the gas sensor (101) using the set of principal parameters by an age compensation modeler (105); and

applying the age compensation model to an output of the gas sensor (101).

2. The method of claim 1, wherein the impedance spectrum for the gas sensor (101) is determined using a potentiostat (102).

3. The method of claim 1 or 2, wherein determining the set of principal parameters comprises modeling an impedance of the gas sensor (101) using a Randles equivalent circuit (300).

4. The method of claim 1, 2 or 3, wherein the set of principal parameters comprise parameters that contribute to the sensitivity of the gas sensor (101) at an amount above a predetermined threshold.

5. The method of claim 1, 2, 3 or 4, wherein a sensitivity of the gas sensor (101) is a function of the electrolyte solution resistance ($R_s$), the double-layer capacitance ($C_{dl}$), the charge transfer resistance ($R_{ct}$) and the Warburg impedance ($W$), and wherein the set of principal parameters comprises one or more of an electrolyte solution resistance ($R_s$), a double-layer capacitance ($C_{dl}$), a charge transfer resistance ($R_{ct}$) and a Warburg impedance ($W$).

6. The method of any preceding claim, wherein the age compensation model is determined by the age compensation modeler (105) as a function of a plurality of principal parameters in the set of principal parameters.

7. The method of any preceding claim, wherein applying the age compensation model to an output of the gas sensor (101) comprises:

determining an output of the age compensation model using the set of principal parameters by the age compensation modeler (105);
generating a signal based on the output of the age compensation model by the age compensation modeler (105); and
adding the signal to an output of the gas sensor (101) by an adder (106).

8. The method of any preceding claim, further comprising detecting an imminent failure of the gas sensor (101), comprising:

detecting a change in one or more of the set of principal parameters by a failure detection module (108); and
issuing a failure warning based on the detected change.

9. The method of claim 8, wherein detecting a change in one or more of the set of principal parameters comprises detecting a structural change in one or more of the set of principal parameters, and wherein detecting the structural change comprises determining whether an estimation error of one or more of the set of principal parameters is higher than a predetermined threshold; or wherein detecting a change in one or more of the set of principal parameters comprises detecting a gross change in one or more of the set of principal parameters, and wherein detecting the gross change comprises determining whether the one or more of the set of principal parameters is higher than a predetermined threshold.

10. A system (100) for age compensation for a gas sensor (101), comprising:

an impedance analyzer (103) configured to determine an impedance spectrum for the gas sensor (101);
a principal parameter identifier (104) configured to determine a set of principal parameters based on the impedance spectrum; and
an age compensation modeler (105) configured to construct an age compensation model for the gas sensor (101) using the set of principal parameters, and to apply the age compensation model to an output of the gas sensor (101).

11. The system (100) of claim 10, wherein the set of principal parameters comprises parameters that contribute to the sensitivity of the gas sensor (101) at an amount above a predetermined threshold.

12. The system (100) of claim 10 or 11, wherein a sensitivity of the gas sensor is a function of the electrolyte solution resistance ($R_s$), the double-layer capacitance ($C_{dl}$), the charge transfer resistance ($R_{ct}$) and the Warburg impedance ($W$), and wherein the set of principal parameters comprises one or more of an electrolyte solution resistance ($R_s$), a double-layer capacitance ($C_{dl}$), a charge transfer resistance ($R_{ct}$) and a Warburg impedance ($W$)

**13.** The system (100) of claim 10, 11 or 12, further comprising an adder (106) configured to add a signal from the age compensation modeler (105) to an output of the gas sensor (101);
wherein the age compensation modeler (105) is further configured to determine an output of the age compensation model using the set of principal parameters, and to generate the signal based on the output of the age compensation model.

**14.** The system (100) of claim 10, 11, 12 or 13, further comprising a failure detection module (108) configured to detect a change in one or more of the set of principal parameters and issue a failure warning for the gas sensor (101) based on the detected change.

**15.** A computer program product comprising a computer readable storage medium containing computer code that, when executed by a computer, implements a method as claimed in any of claims 1 to 9 for age compensation and optionally failure detection for a gas sensor (101).

100

```
┌──────────────┐                      ┌──────────────┐
│    SENSOR    │ ◄═════════════════►  │ POTENTIOSTAT │
│     101      │                      │     102      │
└──────┬───────┘                      └──────┬───────┘
       │                                     ║
       │                                     ▼
       │                              ┌──────────────┐
       │                              │  IMPEDANCE   │
       │                              │   ANALYZER   │
       │                              │     103      │
       │                              └──────┬───────┘
       │                                     │
       │    ┌──────────────────┐      ┌──────▼───────┐
       │    │ FAILURE DETECTION│◄─────│  PRINCIPAL   │
       │    │      MODULE       │      │  PARAMETER   │
       │    │       108         │      │  IDENTIFIER  │
       │    └──────────────────┘      │     104      │
       │                              └──────┬───────┘
       │                                     │
       │                              ┌──────▼───────┐
       ▼        ┌───────────┐◄────────│ AGE COMPEN-  │
      ╱ ╲       │    106    │         │  SATION      │
     ( 106 )◄───│           │         │  MODELER     │
      ╲ ╱       └───────────┘         │     105      │
       │                              └──────────────┘
       ▼
      107
```

# FIG. 1

200

COLLECT SENSOR IMPEDANCE DATA AND DETERMINE IMPEDANCE SPECTRUM
201

DETERMINE PRINCIPAL IMPEDANCE PARAMETERS FROM IMPEDANCE SPECTRUM
202

CONSTRUCT AGE COMPENSATION MODEL USING PRINCIPAL IMPEDANCE PARAMETERS
203

APPLY AGE COMPENSATION MODEL TO SENSOR OUTPUT DURING SENSOR OPERATION
204

# FIG. 2

300

302

301

303 304

FIG. 3

400

COLLECT SENSOR IMPEDANCE DATA AND DETERMINE IMPEDANCE SPECTRUM DURING SENSOR OPERATION
401

DETERMINE PRINCIPAL IMPEDANCE PARAMETERS
402

DETECT STRUCTURAL CHANGE OR GROSS PARAMETER CHANGE IN PRINCIPAL IMPEDANCE PARAMETERS
403

IF SENSOR FAILURE IS DETERMINED TO BE IMMINENT BASED ON STRUCTURAL OR GROSS PARAMETER CHANGE, ISSUE WARNING
404

# FIG. 4

500

PROCESSOR
510

SOURCE
CODE
530

COMPILER
540

INPUT/OUTPUT
DEVICES
570

MEMORY
520

OPERATING SYSTEM
550

APPLICATION
560

# FIG. 5

Europäisches
Patentamt

European
Patent Office

Office européen
des brevets

# EUROPEAN SEARCH REPORT

Application Number

EP 11 25 0176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KITZELMANN D ET AL: "ELECTROCHEMISCHE GASSENSOREN - WIRKUNGSWEISEN UND MOEGLICHKEITEN ZUR FUNKTIONSUEBERWACHUNG. ELECTROCHEMICAL GAS SENSORS- PRINCIPLES AND PERSPECTIVES OF A CONTINUOUS SENSOR CHECK", TECHNISCHES MESSEN TM, R.OLDENBOURG VERLAG. MUNCHEN, DE, vol. 62, no. 4, 1 April 1995 (1995-04-01), pages 152-159, XP000516378, ISSN: 0171-8096 | 1,2,4,6, 8-11,14, 15 | INV. G01N27/02 G01N27/416 |
| Y | * the whole document * ----- | 3,5,12 | |
| Y | FERNANDEZ-SANCHEZ C ET AL: "Electrochemical impedance spectroscopy studies of polymer degradation: application to biosensor development", TRAC, TRENDS IN ANALYTICAL CHEMISTRY, ELSEVIER, AMSTERDAM, NL, vol. 24, no. 1, 1 January 2005 (2005-01-01), pages 37-48, XP004706415, ISSN: 0165-9936, DOI: DOI:10.1016/J.TRAC.2004.08.010 * page 38, column 1 - page 40, column 2; figures 1,2,3 * ----- | 3,5,12 | |
| A | US 2008/000779 A1 (WANG LU [US] ET AL) 3 January 2008 (2008-01-03) * paragraph [0116] - paragraph [0133]; figures 16-20 * ----- | 1-15 | TECHNICAL FIELDS SEARCHED (IPC) G01N |
| A | DE 10 2005 032134 A1 (ENDRESS & HAUSER WETZER GMBH [DE]) 18 January 2007 (2007-01-18) * paragraph [0026] - paragraph [0028] * ----- | 1-15 | |

-/--

The present search report has been drawn up for all claims

| Place of search | Date of completion of the search | Examiner |
|---|---|---|
| Munich | 7 June 2011 | Purdie, Douglas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

# EUROPEAN SEARCH REPORT

Application Number

EP 11 25 0176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 00/14523 A2 (COMINCO LTD [CA]) 16 March 2000 (2000-03-16) * page 6, line 29 - page 7, line 30 * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 7 June 2011 | Purdie, Douglas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 25 0176

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2011

| Patent document cited in search report | | Publication date | Patent family member(s) | | Publication date |
|---|---|---|---|---|---|
| US 2008000779 | A1 | 03-01-2008 | CA | 2696230 A1 | 26-02-2009 |
| | | | EP | 2185923 A1 | 19-05-2010 |
| | | | JP | 2010537198 T | 02-12-2010 |
| | | | WO | 2009026236 A1 | 26-02-2009 |
| DE 102005032134 | A1 | 18-01-2007 | EP | 1899718 A1 | 19-03-2008 |
| | | | WO | 2007006695 A1 | 18-01-2007 |
| WO 0014523 | A2 | 16-03-2000 | AU | 5500499 A | 27-03-2000 |

EPO FORM P0459

For more details about this annex : see Official Journal of the European Patent Office, No. 12/82